# EUROPEAN PATENT APPLICATION

(11) **EP 4 194 562 A1**
(43) Date of publication of application: **14.06.2023**
(21) Application number: 21854623.2
(22) Date of filing: 04.08.2021
(51) Int. Cl.: C12Q 1/02, C12P 21/00, C40B 40/08, C40B 40/10, G01N 33/15

(54) **METHOD FOR SCREENING FOR POLYPEPTIDE THAT ACTS ON TARGET PROTEIN**

(30) Priority: 05.08.2020 JP 2020132823
(71) Applicant: National Institute Of Advanced Industrial Science and Technology, Chiyoda-ku Tokyo 100-8921 (JP)
(72) Inventor: KIMURA Tadashi, Tsukuba-shi, Ibaraki 305-8560 (JP)
(74) Representative: Hoffmann Eitle
(86) International application number: PCT/JP2021/028940
(87) International publication number: WO 2022/030539

(57) **Abstract**

The present invention relates to a method for screening for a polypeptide that acts on a target protein. The present invention relates more specifically, to a method for screening for a polypeptide that acts on a target protein, comprising the steps of:
(1) providing a polynucleotide library constituted of a plurality of expression vectors that can be expressed in a gram-negative bacterium, wherein the plurality of expression vectors each comprise a first polynucleotide encoding a polypeptide different from one another, a secretory signal sequence positioned upstream of the first polynucleotide, and a second polynucleotide encoding a target protein;
(2) transforming a gram-negative bacterium with the expression vector to express the polypeptide in a periplasmic space and the target protein on an inner membrane surface;
(3) contacting the polypeptide with the target protein in the periplasmic space;
(4) allowing the gram-negative bacterium to form a spheroplast to measure activity of the polypeptide on the target protein by an electrophysiological technique; and
(5) identifying a polypeptide that acts on the target protein based on the measured activity.

## Description

### Technical Field

### Related application

This application claims the priority of Japanese Patent Application No. 2020-132823 filed August 5, 2020, the content of which is hereby incorporated by reference.

### Technical field

The present invention relates to a method for screening for a polypeptide that acts on a target protein, a library for the method, a novel method for preparing the library.

### Background Art

An ICK peptide contained in the venom of e.g., poisonous spiders is a polypeptide having three disulfide bonds. The ICK peptide cannot be decomposed by a digestive enzyme. The ICK peptide intravenously administered to rats can be detected in blood over several hours (Non Patent Literature 1).

It is known that an ICK peptide acts on an ion channel to produce its toxicity. The inventors have cloned an ICK peptide that acts on an ion channel and analyzed its characteristics (Non Patent Literatures 2 and 3). If a library of ICK peptides is prepared, a peptide library focusing on the ion channel can be constructed, with the result that peptides that act on various ion channels can be created.

It is known that the ion channel is involved in various diseases (Non Patent Literature 2). Since the stimulation which opens or closes the ion channel is principally, e.g., a change in a membrane potential and in temperature and a mechanical stimulation, a method (strategy) of synthesizing/developing a compound hit in a compound library cannot be employed. Thus, in general, there is a difficulty in promoting development of drug targeting an ion channel.

The inventors conceived a technique, i.e., a PERISS (intra Periplasm Secretion and Selection) method, for searching a peptide that acts on an ion channel or a receptor by using the inner membrane of *Escherichia coli* and the periplasmic space thereof, and are conducting research/development activities (Patent Literature 1). In the PERISS method, peptides of a peptide library focusing on an ion channel are expressed in the periplasmic space of *Escherichia coli,* and allowed to interact with a target molecule expressed on the inner membrane, in the periplasmic space to search a candidate peptide.

To promote development of a drug targeting an ion channel by the PERISS method, it is necessary to confirm that the ion channel is expressed in an active state in the inner membrane of *Escherichia coli.* Whether an ion channel is expressed or not can be confirmed by a conventional technique such as western blotting, but whether the ion channel having a regular three-dimensional structure is expressed in an active state cannot be evaluated by the conventional technique. In the circumstance, the inventors have successfully developed a technique for electrophysiologically measuring the activity of an ion channel forcibly expressed in the inner membrane of *E. coli* by a patch clamp technique (Non Patent Literature 4).

As a method for presenting a molecule in the periplasm of *Escherichia coli,* an anchored periplasmic expression technique is known (Patent Literature 2, Non Patent Literature 5). In the technique, a ligand that can bind to a target molecule is identified through steps: (1) expressing a target molecule in the periplasm of *Escherichia coli,* (2) a micro-hole is formed through the outer membrane of *Escherichia coli,* (3) adding a library of ligands tagged with a fluorescent label in a culture solution, and (4) collecting a ligand bound to the target molecule by a cell sorter with fluorescence used as an index. The anchored periplasmic expression technique is the same as the PERISS method in using the periplasm of *Escherichia coli* as a reaction field but differs in other points.

### Citation List

### Patent Literatures

Patent Literature 1: Japanese Patent Laid-Open No. 2014-207905
Patent Literature 2: WO2005/019409 (National Publication of International Patent Application No. 2006-515514)

### Non Patent Literatures

Non Patent Literatures 1: Kikuchi et al., "High Proteolytic Resistance of Spider-Derived Inhibitor Cystine Knots" International Journal of Peptides (2015) Vol.2015, Article ID 537508
Non Patent Literatures 2: Kimura et al., "Molecular Cloning and Sequence Analysis of the cDNAs Encoding Toxin-Like Peptides from the Venom Glands of Tarantula Grammostola rosea" International Journal of Peptides" (2012) Vol.2012, Article ID 731293
Non Patent Literatures 3: Ono et al., "Characterization of voltage-dependent calcium channel blocking peptides from the venom of the tarantula Grammostola rosea" (2011) Toxicon 58 265-276.
Non Patent Literatures 4: Kikuchi et al., "The application of the Escherichia coli giant spheroplast for drug screening with automated planar patch clamp system" (2015) Biotechnology Reports 7, p17-23
Non Patent Literatures 5: Harvey et al., "Anchored periplasmic expression, a versatile technology for the isolation of high-affinity antibodies from Escherichia coli-expressed libraries" (2004) Proc Natl Acad Sci Vol.101, No.25, p9193-9198.

### Summary of Invention

### Technical Problem

In the conventional PERISS method, a peptide bound to a target molecule is selected by magnetic beads and a part of a library is analyzed by, e.g., a next-generation sequencer. What kind of activity the obtained peptide has must be evaluated by sequencing the DNA of the peptide, producing the peptide encoded by the DNA sequence and evaluating the activity of the peptide. However, if a peptide has a plurality of disulfide bonds within a molecule, like an ICK peptide, chemical synthesis of the peptide requires a costly and time-consuming process and recombinant production of the peptide in *Escherichia coli* is not easy.

An object of the present invention is to provide a method for simply evaluating the activity of candidate peptides obtained by the PERISS method, thereby searching a desired peptide more efficiently, and provide a tool for the method.

### Solution to Problem

To attain the object, the inventors have conducted intensive studies and have found that the interaction between a target protein, which is expressed in the inner membrane of *E. coli* by the PERISS method, and a peptide, which is expressed in the periplasmic space, that is, the action of the peptide on the target protein, can be determined by electrophysiological measurement by a patch clamp technique.

They have also found that ODA analysis is applied to a three-dimensional structure of a ligand for a known membrane protein to predict a site(s) that is likely to interact with other proteins and the sequence of the site(s) is randomized, thereby successfully preparing a library for searching polypeptides that act on various target proteins.

The present invention has been achieved based on the above findings. The following [1] to [15] are provided.
[1] A method for screening for a polypeptide that acts on a target protein, comprising the steps of:
   (1) providing a polynucleotide library constituted of a plurality of expression vectors that can be expressed in a gram-negative bacterium, wherein
      the plurality of expression vectors each comprise a first polynucleotide encoding a polypeptide different from one another, a secretory signal sequence positioned upstream of the first polynucleotide, and a second polynucleotide encoding a target protein;
   (2) transforming a gram-negative bacterium with the expression vector to express the polypeptide in a periplasmic space and the target protein on an inner membrane surface;
   (3) contacting the polypeptide with the target protein in the periplasmic space;
   (4) allowing the gram-negative bacterium to form a spheroplast to measure activity of the polypeptide on the target protein by an electrophysiological technique; and
   (5) identifying a polypeptide that acts on the target protein based on the measured activity.
[2] The method according to [1], wherein the gram-negative bacterium is *Escherichia coli.*
[3] The method according to [1] or [2], wherein the target protein is a membrane protein.
[4] The method according to [3], wherein the membrane protein is a membrane receptor, an ion channel or a transporter.
[5] The method according to any one of [1] to [4], wherein the electrophysiological technique is a patch clamp technique.
[6] The method according to any one of [1] to [5], comprising a step of concentrating the polynucleotide library for the first polynucleotide encoding a polypeptide that binds to a target protein in advance.
[7] A method for producing a polypeptide that acts on a target protein, comprising
   a step of identifying the polypeptide that acts on a target protein by the method according to any one of [1] to [6] and producing the identified polypeptide by gene recombination or chemical synthesis.
[8] A method for preparing a library, comprising the steps of:
   (1) applying an ODA analysis to a three-dimensional structure of a ligand for a known membrane protein to predict a site(s) that is likely to interact with another protein;
   (2) preparing a plurality of polypeptides each having an amino acid sequence in which the predicted site(s) is randomized in the amino acid sequence of the ligand, or preparing a plurality of polynucleotides encoding the polypeptides; and
   (3) preparing a library containing the plurality of polypeptides or the plurality of polynucleotides.
[9] The preparation method according to [8], wherein the ligand is an ICK polypeptide, for example GTx1-15.
[10] The preparation method according to [8] or [9], wherein the membrane protein is a membrane receptor, an ion channel or a transporter.
[11] The method according to any one of [1] to [7], wherein the polynucleotide library is prepared by the method according to any one of [8] to [10]
[12] A library comprising mutually different polypeptides having the following amino acid sequence, or a plurality of polynucleotides encoding the polypeptides,
   DCLGXXRKCIPDNDKCCRPXLVCSRTHKXCXXXX (SEQ ID NO: 1).
[13] The library according to [12], wherein each of the plurality of polynucleotides is inserted in an expression vector that can be expressed in a gram-negative bacterium.
[14] The library according to [13], wherein the expression vector contains the polynucleotide, a secretory signal sequence arranged upstream of the polynucleotide and a polynucleotide encoding a target protein.
[15] A polypeptide having the amino acid sequence set forth in any one of SEQ ID NOs: 4 to 9.

### Advantageous Effects of Invention

According to the method of the present invention, the presence or absence of binding of a polypeptide to a target protein and an interaction such as an inhibitory activity can be determined in a series of steps. The amino acid sequence of the polypeptide selected can be determined by recovering a plasmid (expression vector) from the cell expressing the polypeptide, and analyzing the DNA sequence thereof. More specifically, the polypeptide that acts on a target protein and its activity and sequence information thereof can be simply and quickly obtained.

It is basically difficult to synthesize a peptide, like an ICK peptide, having a plurality of knot-like disulfide bonds in the molecule, in a cell-free system. Although a technique for forming a disulfide bond in a cell-free system by co-expressing, e.g., a molecular chaperone is known, which cysteine residues are bound, cannot be controlled. In a screening method using a cell-free system, peptides in a peptide library and a target molecule are independently expressed in separate systems. For obtaining accurate evaluation when an ion channel is a target molecule, advanced technique by a well-trained operator is required.

In contrast, the method of the present invention employs the periplasmic space of a gram-negative bacterium (e.g., *Escherichia coli)* as a site of expression, a peptide even if it has a complicated three-dimensional structure can be accurately expressed. In the method of the present invention, a library of peptides directing various target molecules and target molecules can be expressed in the same system and simultaneous screening based on an activity can be made. The method of the present invention is useful for screening for a molecule targeting to a membrane molecule, such as a membrane receptor or an ion channel, serving as an important target for developing a drug.

### Brief Description of Drawings

[Figure 1] Figure 1 shows a conceptual diagram of the PERISS method.
[Figure 2] Figure 2 shows a schematic three-dimensional structure of a region involved in the protein-protein interaction, predicted by ODA analysis.
[Figure 3] Figure 3 shows the constitution of a plasmid containing DNA encoding human Kv2.1 and an ODA library.
[Figure 4] Figure 4-1 is a graph showing the results of inhibitory activity of clone 4 against Kv2.1 (control) measured by the patch clamp technique.
Figure 4-2 is a graph showing the results of inhibitory activity of clone 9 against Kv2.1 (control) measured by the patch clamp technique.
Figure 4-3 is a graph showing the results of inhibitory activity of clone 16 against Kv2.1 (control) measured by the patch clamp technique.
Figure 4-4 is a graph showing the results of inhibitory activity of clone 30 against Kv2.1 (control) measured by the patch clamp technique.

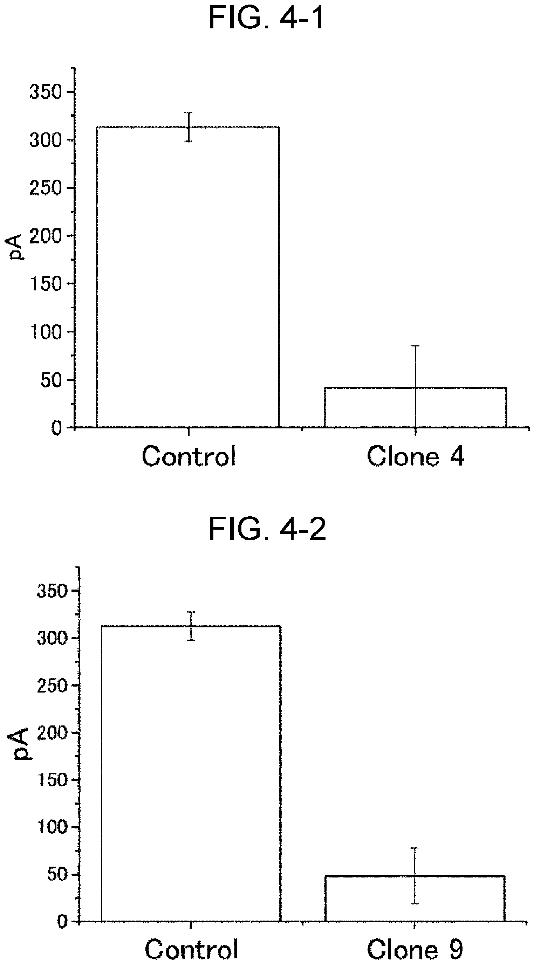

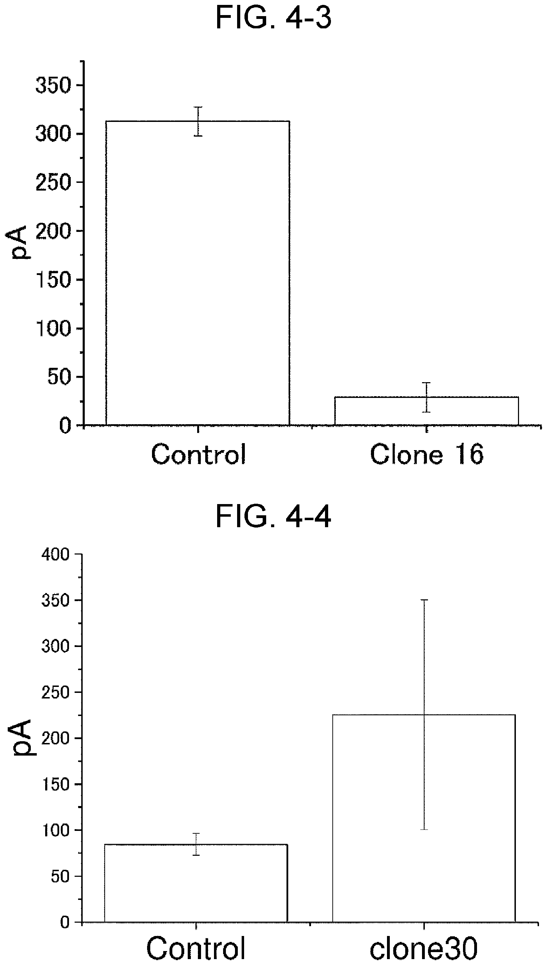

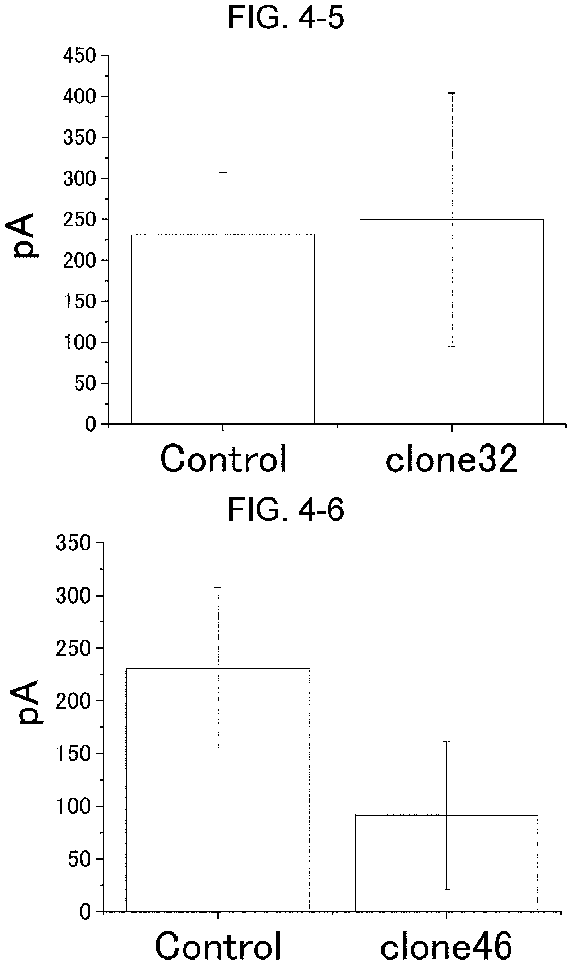

Figure 4-5 is a graph showing the results of inhibitory activity of clone 32 against Kv2.1 (control) measured by the patch clamp technique.
Figure 4-6 is a graph showing the results of inhibitory activity of clone 46 against Kv2.1 (control) measured by the patch clamp technique.

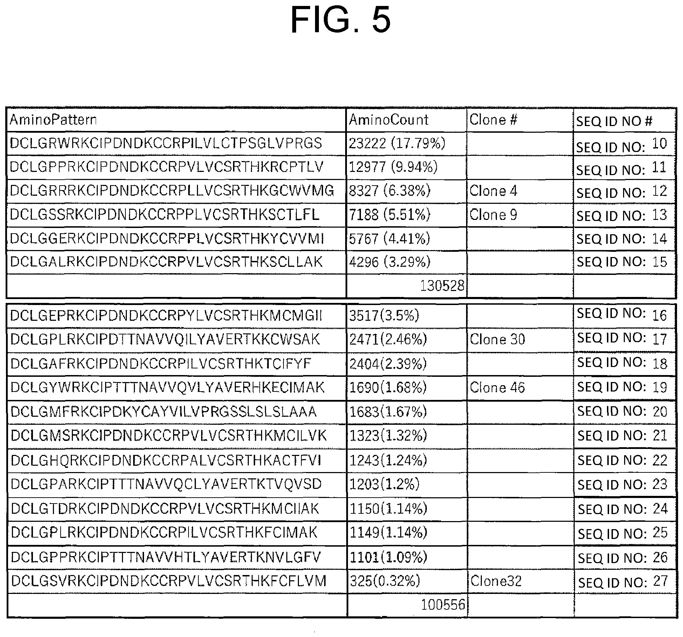

[Figure 5] Figure 5 shows the DNA sequence of a plasmid concentrated five times in accordance with the conventional the PERISS method and analyzed by a next-generation sequencer.

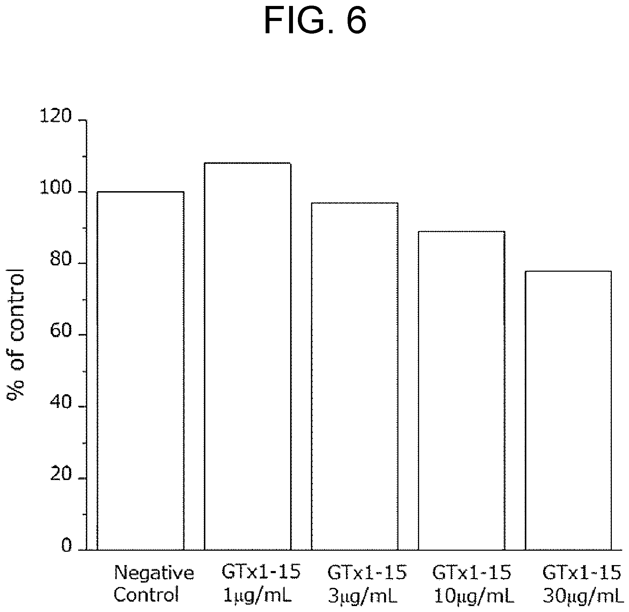

[Figure 6] Figure 6 is a graph showing cytotoxic activity of GTx1-15 against the human monocytic cell line (THP-1).

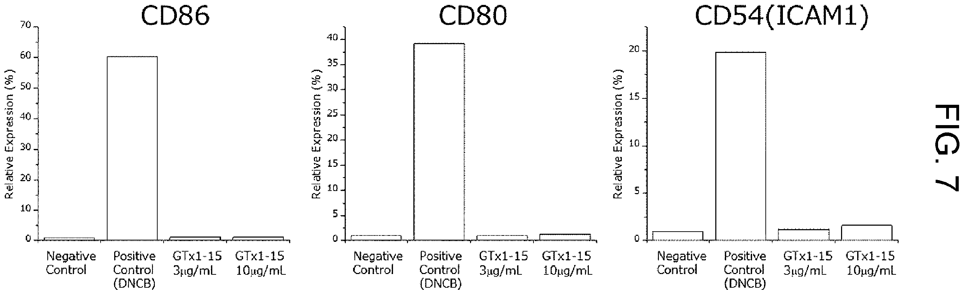

[Figure 7] Figure 7 is a graph showing the immunogenicity of GTx1-15 against the human monocytic cell line (THP-1).

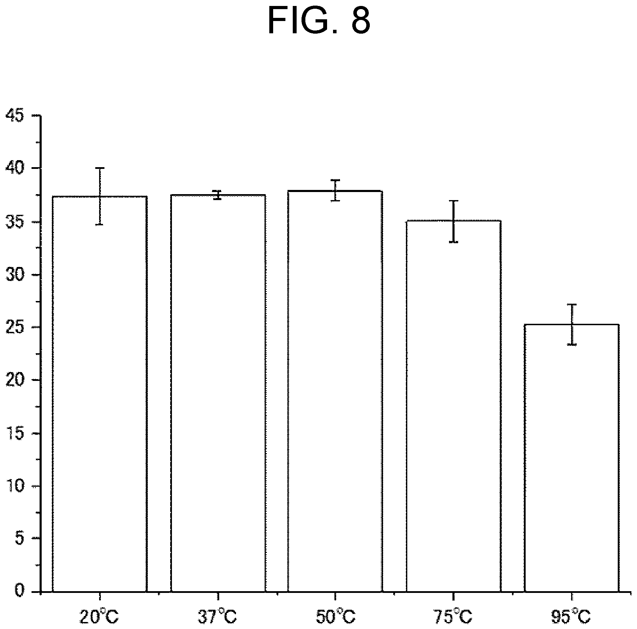

[Figure 8] Figure 8 is a graph showing the temperature stability of GTx1-15.

### Description of Embodiments

### 1. Method for screening for peptide that acts on target protein

### 1.1 Target protein

The present invention relates to a method for screening for a peptide that acts on a target protein.

In the method of the present invention, it is preferable that the "target protein" is a membrane protein. Examples of the membrane protein includes a membrane receptor, an ion channel and a transporter (membrane transporter). In particular, the method of the present invention can be preferably used for screening for a peptide that acts on an ion channel and a transporter, the ligand for which is not known.

Examples of the "membrane receptor" include the following receptors:

### (1) G protein-coupled receptor

The receptor has a seven-transmembrane structure. When a ligand binds to the receptor, G protein is activated to activate or inactivate an enzyme synthesizing a predetermined second messenger, thereby inducing cell response. Examples of the G protein-coupled receptor include a muscarinic acetylcholine receptor, an adrenergic receptor, an adenosine receptor, a GABA_{B} receptor, an angiotensin receptor, a cannabinoid receptor, a cholecystokinin receptor, a dopamine receptor, a glucagon receptor, a histamine receptor, an olfactory receptor, an opioid receptor, a rhodopsin receptor, a secretin receptor, a serotonin receptor, a somatostatin receptor, a gastrin receptor and a P2Y receptor.

### (2) Ionotropic receptor

When a ligand binds to the receptor, the structure of the receptor changes, which enables a predetermined ion to pass through the ion channel to change the membrane potential. Examples of the ionotropic receptor include a nicotinic acetylcholine receptor, a glycine receptor, a GABA receptor (GABA_{A} and GABA_{C}), a glutamate receptor, a serotonin receptor (type 3), P2X receptor, a voltage-gated ion channel (receptor) that acts in response to the membrane potential of other cells.

### (3) Cytokine receptor superfamily

When a ligand binds to the receptor, the receptor changes into a dimer and is activated by interaction with tyrosine kinase. Examples of the cytokine receptor include various cytokine receptors and a prolactin receptor.

### (4) Receptor having enzymatic activity

When a ligand binds to the receptor, tyrosine kinase activity and guanylate cyclase activity of the cytoplasm are enhanced. Examples of the receptor having enzymatic activity include receptors for many growth factors, such as a tyrosine kinase receptor, a guanylate cyclase receptor and an insulin receptor.

The "ion channel" refers to a transmembrane protein present in the biological membrane of a cell and passively passing ions therethrough. The ion channel is related to various physiological functions in which ions are involved, such as activity of neural circuits, muscle contraction and interval. Ion channels are classified bases on ion selectivity (e.g., potassium channel, sodium channel, calcium channel, cation channel), and gate opening/closing control (voltage-gated, ligand-gated, mechanical stimulus-gated, temperature-gated, phosphorylation-gated controls).

Examples of the ion channel include the following ones.

### (1) Voltage-gated channel

Examples of the voltage-gated channel include a voltage-gated potassium channel, a voltage-gated sodium channel, a voltage-gated calcium channel, a calcium-activated potassium channel, an HCN channel, CNG channel, a TRP channel and a voltage-gated proton channel.

### (2) Inward rectifier potassium channel

Inward rectifier potassium channels are roughly classified into a classic channel, such as Kir1, 2, 4, 5 and 7, Kir3 to be activated by a GTP binding protein, and Kir6 constituting an ATP sensitive potassium channel. The inward rectifier potassium channel plays an important role in maintaining the resting membrane potential of nerve cells and myocardium.

### (3) Ligand-gated channel

Examples of the ligand-gated channel include a Cysloop receptor (glycine receptor, GABA receptor (GABA_{A}, GABA_{C})), a glutamate receptor and P2X receptor. These serves also as membrane receptors.

### (4) Intracellular membrane system ion channel

Examples of the intracellular membrane system ion channel include a receptor-type ion channel present in the intracellular membrane such as a ryanodine receptor, IP3 receptor and a TRIC channel.

### (5) Others

Examples of the other ion channels include a two-pore channel, an acid-sensitive ion channel, an epithelial sodium channel, a chlorine channel and connexin.

The "transporter (membrane transporter)" refers to a protein passing through a biological membrane and responsible for transferring a substance through the membrane. Examples of a transporter in humans include two super families: a SLC (solute carrier) transporter and an ABC (ATP-binding cassette) transporter, and other several families.

The membrane receptor, ion channel and transporter functions are mutually overlapped. These membrane proteins are included in the "target protein" of the present invention as long as activation thereof can be detected by an electrophysiological technique to be applied to cells (cell membrane), regardless of their names.

### 1.2 Individual steps of screening method

The screening method of the present invention includes the following steps:
Step 1: providing a library of polynucleotides consisting of a plurality of expression vectors that can be expressed in a gram-negative bacterium, wherein
   the plurality of expression vectors each containing
   a first polynucleotide encoding a polypeptide different from the others, a secretory signal sequence arranged upstream of the first polynucleotide, and a second polynucleotide encoding a target protein;
Step 2: transforming a gram-negative bacterium with the expression vector to express the polypeptide in a periplasmic space and the target protein on an inner membrane surface;
Step 3: contacting the polypeptide with the target protein in the periplasmic space;
Step 4: allowing the gram-negative bacterium to form a spheroplast and measuring the activity of the polypeptide on the target protein by an electrophysiological technique; and
Step 5: identifying a polypeptide that acts on the target protein based on the measured activity.

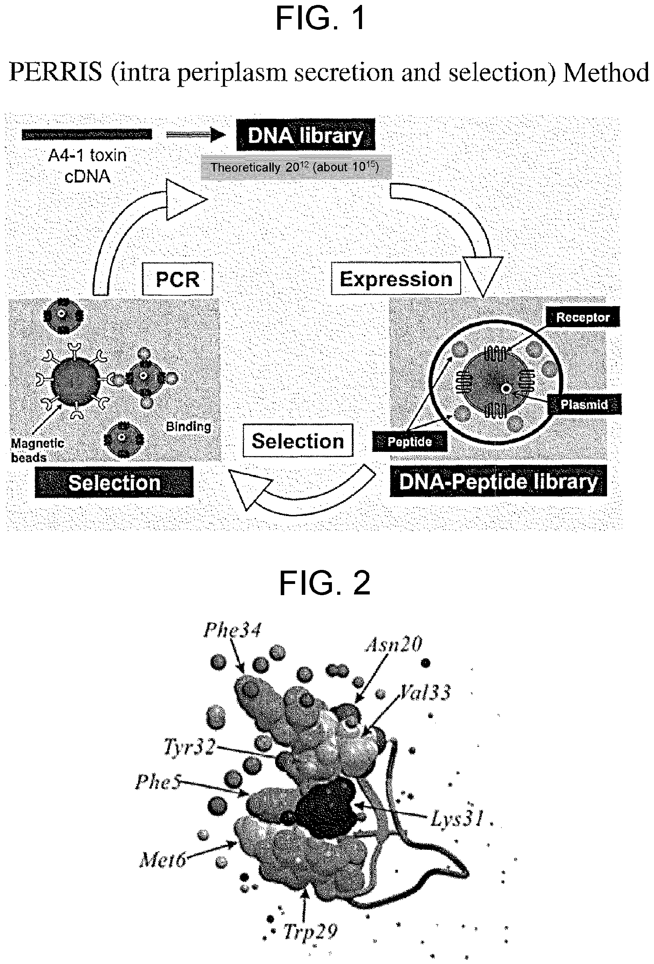

Now, individual steps of the present invention will be more specifically described below. Of these steps, Step 1 to Step 3 are carried out in accordance with the PERISS method. The PERISS method is schematically shown in Figure 1.

### (Step 1) Providing polynucleotide library

First, there is provided a polynucleotide library constituted of a plurality of expression vectors that can be expressed in a gram-negative bacterium.

In the present invention, the "expression vector" is not particularly limited as long as it can be expressed in a gram-negative bacterium and may be a vector that can be expressed in other microorganisms and a cell-free system. Each of the expression vectors contains a first polynucleotide encoding a polypeptide different from one another, a secretory signal sequence, and a second polynucleotide encoding a target protein. The expression vector is preferably a plasmid containing an expression cassette for the first polynucleotide and an expression cassette for the second polynucleotide.

The "first polynucleotide" contains a randomized sequence portion constituting a library; and therefore, each expression vector encodes a different polypeptide.

The "secretory signal sequence" is operably connected to upstream of the first polynucleotide to allow a polypeptide, which is encoded by the first polynucleotide and expressed in a bacterial cell, to secrete outside the inner membrane (in the periplasmic space). The periplasm refers to a space surrounded by two biological membranes, i.e., inner membrane and outer membrane, in a gram-negative bacterium. The secretory signal sequences are in common in all expression vectors.

The "second polynucleotide" encodes a target protein and is identical in all expression vectors. The second polynucleotide is integrated into an expression vector such that the target protein is expressed on the inner membrane of a gram-negative bacterium. In the case where a target protein is a soluble protein (e.g., enzyme) other than the membrane protein, the target protein is expressed such that it connects to a single transmembrane protein at the periplasmic side.

The expression vector is designed such that the first polynucleotide is expressed in the periplasmic space and the second polynucleotide is expressed on the inner membrane. Such a design of an expression vector is already known in the technical field. For example, to the upstream side (5' side) of the first polynucleotide, not only the secretory signal but also a tag sequence, an SD sequence and a promoter sequence are added in order to facilitate collection/purification of a polynucleotide in the later step, and a stop codon is added to the downstream side (3' side) thereof. To the upstream side (5' side) of the second polynucleotide, an SD sequence and a promoter sequence are added, and a stop codon is added to the downstream side (3' side) thereof.

In the present invention, the "gram-negative bacterium" is not particularly limited. Examples of the gram-negative bacterium that can be used include *Escherichia coli, salmonella* of the *proteobacteria phylum, pseudomonas,* and acetobacter; cyanobacterium, and green sulfur bacterium. Typically, *Escherichia coli* is used. Individual codons of a polynucleotide can be optimized such that the polypeptide encoded by the polynucleotide can be efficiently expressed in a host gram-negative bacterium.

For example, a method for preparing a typical expression vector (plasmid) for *Escherichia coli* will be described. To the 5' side of the first polynucleotide, a signal sequence (e.g., OmpA) for transporting a polypeptide to the periplasmic space of *Escherichia coli,* followed by a tag sequence (e.g., Strept-tag (registered trademark)) for purification are arranged; and further, a regulatory sequence including a promoter sequence is added so as to express the polypeptide as a fusion protein with a periplasmic interstitial enzyme (e.g., DsbC) of *Escherichia coli;* and a stop codon is added to the 3' side thereof. Fusion with a periplasmic interstitial enzyme enhances solubility of the polypeptide and enables stable expression of the polypeptide in the periplasmic space.

### (Step 2) Transformation of gram-negative bacterium and expression of polypeptide

A gram-negative bacterium is transformed with the above expression vector to express polypeptides encoded by the first and second polynucleotides. The gram-negative bacterium is transformed by a technique commonly used in this field such as electroporation and a calcium chloride method.

The polypeptide encoded by the first polynucleotide is transcribed/translated, and thereafter, secreted in the periplasmic space by the help of the secretory signal (sequence). Since a single type of polynucleotide contained as the first polynucleotide in a single expression vector is introduced in a single cell, the single polypeptide is secreted in the periplasmic space. In contrast, the target protein encoded by the second polynucleotide is identical in all cells and expressed on the inner membrane.

### (Step 3) Contact between polypeptide and target protein

In the periplasmic space, the polypeptide encoded by the first polynucleotide is placed in a state where it can be in contact with a target protein expressed in the inner membrane (Figure 1). Based on the same principal as that underlying the PERISS method, a polypeptide having an affinity for a target protein, binds to the target protein on the inner membrane to form a complex.

### (Step 4) Measurement of activity of polypeptide on target protein

A gram-negative bacterium is converted into a spheroplast and activity of the polypeptide on the target protein is measured by an electrophysiological technique.

The "spheroplast" (spherical cell) is a microbial cell from which the cell wall has been partially removed. A gram-negative bacterial cell is treated with, e.g., lysozyme or penicillin to obtain a spheroplast. Since cell membrane of the spheroplast of a gram-negative bacterium is partially removed, interaction (electrophysiological change) between a target protein and a polypeptide can be analyzed by an electrophysiological technique. In a single spheroplast, a single type of polypeptide is present in a high concentration. This is a favorable condition for measuring activity of the polypeptide on a target protein.

As the "electrophysiological technique", a patch clamp technique can be used. The patch clamp technique is a technique for directly measuring the activity of an ion channel or transporter on the cell membrane by recording ionic behavior via the ion channel or a transporter. In the patch clamp technique, a high resistant seal is formed between a glass electrode and a cell membrane. By virtue of this, the potential from a micro cell as small as an electrode tip can be measured and a micro current mediated by e.g., ion channel, on the cell membrane, can be measured. Various techniques are known as the patch clamp technique, such as a cell attach technique and a whole cell technique. Those skilled in the art appropriately select a technique from these depending on the purpose, and put in use.

As the "electrophysiological technique", a two-electrode potential clamp method using cultured cells of Xenopus oocytes or CHO, can be used. For example, a peptide is allowed to bind to a single transmembrane protein on a periplasm side and expressed. In this manner, the peptide can be expressed while being anchored on cell membrane. When the ion channel is expressed in a xenopus oocyte in accordance with this method; at the same time, a peptide is expressed while being anchored on the cell membrane of the oocyte, it is possible to measure the activity by the two-electrode potential clamp method. According to this method, it is considered that measurement can be more efficiently carried out compared to measurement using a free peptide. This technique is generally called as a membrane-tethered toxin method (Ibanez-Tallon et al. Neuron, 43, 305-311, 2004).

Although the activity measurement varies depending on the characteristics of, e.g., a target protein, the activity can be measured by comparing a change in current value measured when the resting membrane potential changes from, for example-80 mV to +20 mV, or a change in current value measured when the resting membrane potential changes from, for example -80 mV to +60 mV in steps of +10 mV, to that of a negative control where a polypeptide is not expressed.

In conventional PERISS method, binding between a target protein and a polypeptide in the periplasmic space can be evaluated, but the activity (agonist activity or antagonist activity) of a polypeptide on a target protein cannot be evaluated. In the method of the present invention, an electrophysiological change of a cell caused by interaction of a polypeptide on a target protein is measured by converting a gram-negative bacterial cell into a spheroplast. In this manner, the activity of a polypeptide on a target protein can be directly evaluated.

### (Step 5) Identification of polypeptide that acts on target protein

Finally, a polypeptide that acts on a target protein is identified based on the activity measured by an electrophysiological technique.

For example, when the target protein is an ion channel, if current flow via the ion channel is significantly decreased by binding of a polypeptide, it can be identified (determined) that the polypeptide has an antagonist activity to the target protein. In contrast, if current flow via the ion channel is significantly increased by binding of a polypeptide, it can be identified (determined) that the polypeptide has an agonist activity to the target protein. The measured activity has one-to-one correspondence between the polypeptide and the polynucleotide sequence encoding the polypeptide, as described above. Accordingly, if an expression vector (plasmid) is purified from a cell having a desired activity and the sequence of the vector is determined by, e.g., the Sanger method, the amino acid sequence of the desired polypeptide can be identified. It is known that a transporter also passes a minute electric current, so that the activity can be measured by inhibiting the electric current. In the case of a membrane receptor such as GPCR, if an ion channel such as Kir is connected to the membrane receptor on the C terminal side, a structural change of the receptor can be measured as the activity of an ion channel.

### 1.3 Concentration of library

The library used in the present invention is preferably concentrated in advance for (before) binding of a target protein. The library can be concentrated by repeating a process of selecting/amplifying a polynucleotide encoding a polypeptide bound to a target protein in accordance with step 1 to step 3, referring to the previous reports (WO2010/104114, Japanese Patent Laid-Open No. 2014-207905).

More specifically, a cell containing a polypeptide bound to a target protein is selected, and thereafter, a polynucleotide encoding the polypeptide contained in the cell is recovered by, e.g., a miniprep method, and amplified to prepare a second library.

A polypeptide bound to a target protein is recovered by a typical method which comprises converting a gram-negative bacterial cell into a spheroplast and selecting the polypeptide by use of beads (e.g., Strep-Tactin (registered trademark) beads) that binds to a tag (for example, Strept-tag (registered trademark)) previously introduced in the polypeptide. Examples of the tag that can be used herein include streptavidin and improved streptavidin protein (Strept-tag (registered trademark)), T7, histidine, and FLAG tag. The polypeptide can be selected by use of magnetic beads, sepharose beads, agarose beads, porous beads, a plate or a membrane.

From the selected spheroplast cell of the "polypeptide-membrane protein-spheroplast complex" immobilized onto the beads, plasmid DNA is isolated. Then, the sequences of polynucleotides constituting a polynucleotide library in the plasmid DNA are amplified by PCR to prepare a second library. The second library has the same randomized region and framework region as those of an original library but the affinity of the whole library for a target protein is increased and the degree of sequence variation in the randomized region should have decreased ("concentrated"). If this step is repeated, that is, many rounds of selection and generation (construction) of a library are carried out, an assembly (concentrated library) of polynucleotides encoding a polypeptide having higher affinity for the target protein can be obtained (see, Figure 1).

After the second round that is the round for selection, polypeptides can be concentrated for a polypeptide with a higher binding affinity by sequentially increasing selection pressure, for example, by reducing the incubation time of a complex of a polypeptide-membrane protein-spheroplast, by increasing the number of washing times and/or increasing the temperature.

The number of concentration steps, which varies depending on the structure or size of a library, is usually 2 to 10 rounds, preferably 3 rounds or more (for example, 3 to 8 rounds), more preferably 5 rounds or more (for example, 5 to 7 rounds).

### 2. Method for producing polypeptide that acts on target protein

The polypeptide that acts on a target protein identified as mentioned above can be produced by gene recombination or chemical synthesis depending on the structure thereof. Such a method for producing a polypeptide that acts on a target protein falls within the range of the present invention.

### 3. Method for producing library using ODA analysis

The present invention provides a method for producing a library using the ODA (Optimal Docking Area) analysis.

The "ODA analysis" refers to a method for predicting a protein-protein interaction region in a predetermined protein based on desolvation energy (Juan Fernand es-Recio et al. "Optimal Docking Area: A New Method for Predicting Protein-Protein Interaction Sites" POTEINS: Structure, Function, and Bioinformatics 58: 134-143 (2005)). In the present invention, the region predicted by ODA analysis as having a small desolvation energy value in the surface of a predetermined protein is determined as a site(s) where protein-protein interaction may occur (site(s) that is likely to interact with another protein). Then, a polypeptide library consisting of a plurality of polypeptides in which the site(s) is randomized is prepared. Also, a polynucleotide library consisting of polynucleotides encoding the polypeptides is prepared.

To describe more specifically, a method for producing a polypeptide library according to the present invention includes the following steps:
(1) predicting a site that is likely to interact with another protein by applying the ODA analysis to a three-dimensional structure of a ligand for a known membrane protein;
(2) preparing a plurality of polypeptides each having amino acid sequences in which the predicted site(s) is randomized in the amino acid sequence of the ligand; and
(3) preparing a polypeptide library consisting of a plurality of polypeptides.

A method for producing a polynucleotide library according to the present invention includes the following steps:
(1) predicting a site(s) that is likely to interact with another protein by applying the ODA analysis to a three-dimensional structure of a ligand for a known membrane protein;
(2) designing a plurality of polypeptides each having an amino acid sequence of the ligand in which the predicted site(s) is randomized in the amino acid sequence of the ligand, and preparing a plurality of polynucleotides encoding the polypeptides; and
(3) preparing a polynucleotide library containing the polynucleotides.

As the membrane protein, a membrane receptor, an ion channel, or a transporter as mentioned in Section 1.1 can be used. The membrane protein is preferably a membrane receptor or an ion channel.

A ligand for a membrane protein already known is not particularly limited as long as it has a structure of a polypeptide commonly known. As a preferable example of a ligand, an ICK polypeptide.

The ICK polypeptide refers to a series of polypeptides having an intrinsic structural motif called as an inhibitor cystine knot (ICK) contained in a spider venom component. The structural motif contains 6 cysteine residues (Cys-1 to Cys-6) and Cys-1 and Cys-4, Cys-2 and Cys-5, and Cys-3 and Cys-6, are connected via an S-S bond to form knot-like cysteine bonds. In the ICK polypeptide, the structural motif is strictly conserved but other sequences except the motif vary. As previously mentioned, since it is difficult to express a peptide having a complicated structure like an ICK polypeptide in a cell-free system, such a peptide is suitable for the screening system of the present invention. Examples of the ICK peptides include, but are not limited to, those shown in the following Table.

**[Table 1]**

| **Name of polypeptide having ICK skeleton** | **Data base/Accession No.** |
|---|---|
| Ceratotoxin-1 (CcoTx1) | P84507 |
| Ceratotoxin-2 (CcoTx2) | P84508 |
| Ceratotoxin-3 (CcoTx3) | P84509 |
| Covalitoxin-2 (CvTx-II) | P82601 |
| Hainantoxin-1 (HnTx-I) | P83591 |
| Hainantoxin-3 (HnTx-III) | P83464 |
| Hainantoxin-4 (HnTx-IV) | P83471 |
| Hainantoxin-5 (HnTx-V) | P60975 |
| Hanatoxin-1 (HaTx1) | P56852 |
| Hanatoxin-2 (HaTx2) | P56853 |
| Heteroscodratoxin-1 (HmTx1) | P60992 |
| Heteroscodratoxin-2 (HmTx2) | P60993 |
| Huwentoxin-1 (HwTx-1) | P56676 |
| Huwentoxin-4 (HwTx-IV) | P83303 |
| Omega-grammotoxin SIA (Omega-GsTx SIA) | P60590 |
| Phrixotoxin-3 (PaurTx3) | P84510 |
| Protoxin-1 (ProTx1) | P83480 |
| Protoxin-2 (ProTx2) | P83476 |
| Psalmotoxin-1 (PcTx1) | P60514 |
| Stromatoxin-1 (ScTx1) | P60991 |
| Theraphotoxin-1 (TITx1) | P83795 |
| Toxin GsMTx-4 (GsMTx4) | Q7YT39 |
| Toxin SNX-482 (SX482) | P56854 |
| Toxin 5GTx1 (SGTx1) | P56855 |
| Venom protein 5 (TXP5) | P49266 |
| Voltage sensor toxin 1 (VSTX1) | P60980 |
| Voltage sensor toxin 2 (VSTX2) | P0C2P4 |
| Voltage sensor toxin 3 (VSTX3) | P0C2P5 |

GTx series can be preferably used (mentioned above, Kimura, et al., 2012). Of the GTx series, GTx1 and GTx2 are ICK peptides. Typical examples of GTx3 include MIT1 and Prokineticin. GTx4, GTx5 and GTx6 are disulfide-rich peptides having a plurality of SS bond in a molecule. GTx7 is a cyclic peptide. Another peptide such as a conotoxin isolated from corn snail can be used.

The three-dimensional structure of a ligand can be determined based on the information commonly available or by use of NMR or predicted by homology modeling. The method for obtaining information on the three-dimensional structure is appropriately selected depending on the size and structure of the ligand.

In the method of the present invention, a site(s) that is likely to interact with another protein is predicted from the three-dimensional structure of a ligand by the ODA analysis and produce a library (of polypeptides) in which the site(s) is randomized. The method of the present invention enables to efficiently screen for not only an original target molecule (a membrane protein known in this field) but also other polypeptides that act on a target protein. Accordingly, the method of the present invention can be used for searching a ligand for an orphan receptor.

The library prepared by the method of the present invention can be preferably used not only in the screening method of the present invention but also in other screening methods such as a system (method) using a cell-free system.

The library thus prepared may be concentrated by a method known in the technical field depending on the purpose. The concentration step, for example, if the PERISS method is applied, is usually carried out 2 to 10 rounds, preferably 3 rounds or more (for example, 3 to 8 rounds), more preferably 5 rounds or more (for example, 5 to 7 rounds). If the sequence of a polypeptide is selected by screening using *in vitro* molecular evolution, a part of the sequence is further randomized to prepare a library of offspring thereof.

### 4. Polypeptide library and polynucleotide library of the present invention

The inventors prepared a polypeptide library and a polynucleotide library based on the structure of GTx1-15 (a kind of ICK peptide) in accordance with the method of the present invention. The present invention also provides these libraries.

The polypeptide library based on the structure of GTx1-15 contains mutually different polypeptides having the following amino acid sequence: DCLGXXRKCIPDNDKCCRPXLVCSRTHKXCXXXX (SEQ ID NO: 1).

The polynucleotide library based on the structure of GTx1-15 contains mutually different polynucleotides encoding polypeptides having the amino acid sequence (SEQ ID NO: 1).

The polynucleotide library of the present invention is expressed as polypeptides by introducing it into an appropriate expression vector and transforming a host cell with the vector. As mentioned above, the library of the present invention can be used in a screening method in a cell-free system and in other systems.

When the polynucleotide library of the present invention is used in the PERISS method or the screening method of the present invention, a plurality of polynucleotides serving as members of the library are inserted respectively into expression vectors that can be expressed in a gram-negative bacterium. At this time of insertion, other than a polynucleotide as mentioned above, a secretory signal sequence, and a polynucleotide encoding a target protein are contained in an expression vector. The expression vector can be designed based on common technical knowledge and the description of Sections 1 and 2.

### 5. Use of polypeptide that acts on target protein, obtained by the screening (method) of the present invention

The polypeptide obtained by the screening method of the present invention can be used for developing a drug targeting a (target) protein, and used as a detection reagent for detecting a target protein in a sample or an affinity carrier for purifying a target protein.

A preferable target protein in the present invention is a membrane protein involved in transduction of various signals on biological membrane surface. Accordingly, the polypeptide obtained by the screening method of the present invention is useful for searching not only a target protein including various membrane receptors, drug transporters, agonists and antagonists such as ion channel but also a ligand substance to an orphan receptor. It is expected that the polypeptide obtained by the screening (method) of the present invention may have a pharmacological action used alone for, e.g., a therapeutic agent or a diagnostic agent and may provide a lead compound for developing drugs in future.

Examples of the polypeptide obtained by the screening method of the present invention include polypeptides set forth in SEQ ID NOs: 4 to 9. Of these polypeptides, the polypeptides set forth in SEQ ID NO: 4 to 7 and 9 were selected/identified as those having an agonist or antagonist activity on human Kv2.1 serving as a potassium channel from the library designed based on the skeleton of GTx1-15. The polypeptide set forth in SEQ ID NO: 8 was selected/identified as that having neither one of the activities. Potassium channel Kv2.1 present in β-cells of the pancreas is a delayed rectification channel which returns the excited state of a cell membrane to the original state (resting membrane potential). If the potassium channel Kv2.1 is inhibited, it is known that the excited state of cells is extended and insulin release from β-cells can be augmented. The peptide having an antagonist activity can be expected to serve as an antidiabetic drug. Since it is also known that expression of Kv2.1 is augmented in stomach cancer and uterine cancer, the peptide having an antagonist activity can serve as a therapeutic drug for stomach cancer and uterine cancer. In contrast, the peptide having an agonist activity such as an activity to induce apoptosis by activating Kv2.1 of medulloblastoma can be expected to serve as a therapeutic drug for suppressing proliferation of cancer cells. A peptide exhibiting neither one of the activities is available as a binder.

### Examples

Now, the present invention will be more specifically described by way of Examples, below. The present invention is not limited to these Examples.

### Example 1: Preparation of peptide library

A peptide library for searching a polypeptide that specifically binds to a target membrane protein was prepared by applying the ODA analysis to an ICK peptide, GTx1-15 (SEQ ID NO: 2). The ODA analysis is a technique of predicting a site of a peptide or protein molecule to interact with another molecule based on desolvation (Proteins. 2005 Jan 1; 58 (1): 134-43). In the image obtained by the analysis, the size of the "sphere" corresponds to its energy value. A large size "sphere" represents a small energy value and is considered to be highly related with the protein-protein interaction.

The inventors applied the ODA analysis to the three-dimensional structure of GTx1-15 predicted by homology modeling to specify the site highly related with the protein-protein interaction in GTx1-15 (Figure 2, Ono et al. 2011). The site may be expected to likely interact also with a protein other than a target molecule. Accordingly, if a library for this site is formed, it is considered that a peptide targeting a different molecule from the original target molecule of the peptide can be screened for.

The, the site identified was randomized to prepare a peptide library for searching a polypeptide. The amino acid sequence of the resultant peptide library obtained is as follows:
DCLGXXRKCIPDNDKCCRPXLVCSRTHKXCXXXX (SEQ ID NO: 1).

### Example 2: Screening for peptide that binds to target protein by the PERISS method

DNA (SEQ ID NO: 3) encoding a target protein, human Kv2.1, was integrated into a plasmid. *Escherichia coli* C43 (DE3) strain was transformed with the plasmid and developed (cultured) in LB agar medium to obtain colonies. A single colony was picked up to prepare a huge spheroplast. The current generated was measured by the patch clamp technique in accordance with a report previously published. As a result, it was confirmed that a current of (human) Kv2.1 can be measured (Kikuchi et al. Biotechnology Reports, 2015).

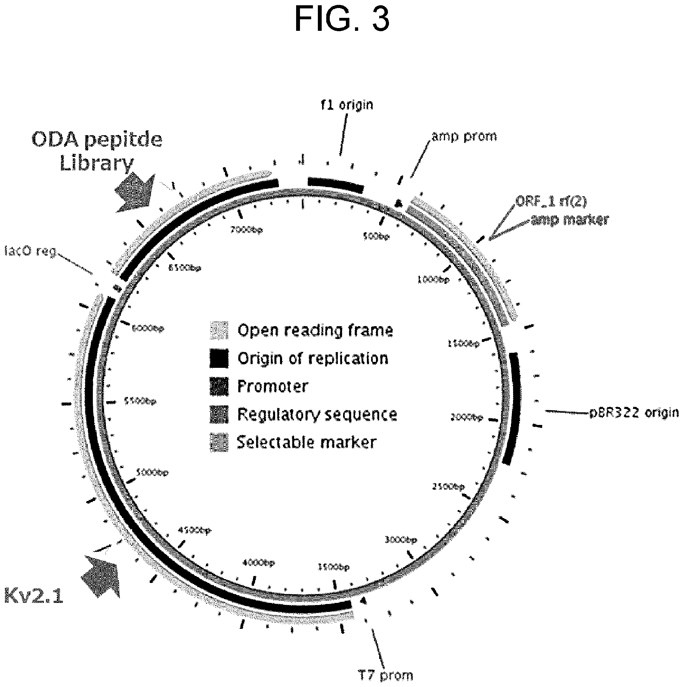

To the plasmid, peptides of the library prepared in Example 1 were integrated tandemly to prepare a construct (peptide library plasmid DNA) so as to express a target membrane protein and the peptide of the library simultaneously from a single plasmid (Figure 3). As shown in Figure 3, to the 5' side of the DNA sequence encoding the ODA peptide of the library, a signal sequence OmpA, and subsequently a Strept-tag (registered trademark) for purification were arranged, and then, a sequence encoding DsbC (periplasmic interstitial enzyme of *Escherichia coli)* was added via a linker.

A PERRIS method was applied to the peptide-library plasmid DNA prepared to search a peptide that binds to a target protein (human Kv2.1), as follows.

To competent cells (100 µL) of *E. coli* C43 (DE3) strain, the peptide library plasmid DNA was added and the cell suspension was allowed to stand still on ice for 3 minutes. Subsequently, the cell suspension was transferred to a cuvette for electroporation and electroporated to introduce the plasmid DNA into the cell strain. The cell suspension (100 µL) containing the cells electroporated was suspended in SOC (1 mL) medium, which was then transferred to a 15-mL tube and (recovery-) cultured at 37°C and 200 rpm for one hour. The total amount of the (recovery-)culture medium was smeared onto an LB plate (containing 50 µg/ml ampicillin). Thereafter, culture was carried out at 37°C for about 16 hours. After completion of the culture, an LB medium (containing 50 µg/ml ampicillin) was added dropwise onto the surface of the LB plate medium in an amount of 3 mL per plate and sufficiently suspended with bacterial cells. The cell suspension was inoculated in a 100 ml LB medium (containing 50 µg/ml ampicillin) contained in a 500 ml Erlenmeyer flask, and cultured at 37°C and 200 rpm. One hour after initiation of the culture, 100 µl of IPTG was added (final concentration: 1 mM) and the culture was continued at an incubation temperature of 25°C and a rotation speed of 200 rpm. About 3.5 hours after initiation of the culture, a culture was transferred to centrifuge tubes and centrifuged at room temperature in the condition of 2000 g for 5 minutes. After centrifugation, the cells were converted into spheroplasts in accordance with a routine method. In 1 mL of 0.6 M sucrose buffer (750 µL of 0.8 M sucrose, 20 µL of 1M Tris-HCl, 230 µL of H₂O, 5 mg of BSA), the spheroplasts were suspended. To the spheroplast suspension, streptavidin magnetic beads were added. The bead/spheroplast suspension solution was gently shaken at room temperature for 20 minutes. Using a magnetic stand, magnetic beads were washed twice with 1 mL of 0.6 M sucrose buffer (15 mL of 0.8 M sucrose, 0.6 mL of 5 M NaCl, 0.4 mL of 1 M Tris-HCl, 2 mL of H₂O). After completion of washing, the spheroplast cells were recovered and plasmids were purified from the spheroplast cells.

In order to concentrate the peptides that bind to a target protein, the purified plasmid was added to competent cells (100 µL) of *E. coli* C43 (DE3) strain and the PERISS method (more specifically, a step of concentrating plasmids expressing a peptide that binds to a target protein) was applied repeatedly four times in the same manner as above. The PERISS method was applied five times in total.

### Example 3: Determination of activity of peptide contained in library concentrated

The activity of a peptide contained in the library which have been concentrated for a peptide that binds to a target protein by repeating the PERISS method 5 times, was determined by a patch clamp technique.

After the concentration, C43 (DE3) *Escherichia coli* strain was transformed with the plasmid. *E. coli* C43 (DE3) library plasmid-introduced strain was smeared (streaked) onto a 2% agar plate of an LB medium containing a 50 µg/ml ampicillin and subjected to static culture at 37°C for 24 hours. After completion of the static culture, a single colony on the culture plate was picked up, inoculated in 10 ml of an LB medium (containing 50 µg/ml ampicillin) contained in a 100 ml-Erlenmeyer flask and pre-cultured (shaking culture) at 37°C and 200 rpm for 18 hours. After the pre-culture, 1 ml of the pre-culture solution was inoculated in a 100 ml-LB medium, which contained 100 µl of 50 µg/ml ampicillin and 600 µl of 10 mg/ml Cephalexin, was dispensed in a 500 ml Erlenmeyer flask, and subjected to a main culture (shaking culture) at 37°C and 200 rpm. One hour after initiation of the main culture, 100 µl of 1 M IPTG was added (final concentration: 1 mM) and culture was continued by changing an incubation temperature to 30°C at 200 rpm. At the time point when O.D.600 reached 0.2 to 0.4, the culture was transferred to centrifuge tubes and subjected as samples for spheroplast preparation. Transfer to the centrifuge tubes was carried by transferring 45 ml of the culture solution to a 50 ml-tube. The *Escherichia coli* cells grown were collected and the supernatant was removed. The *Escherichia coli* cells were converted into huge spheroplasts. Electrophysiological measurements of the huge spheroplasts were carried out by Port-a-patch device manufactured by Nanion. While the resting membrane potential was fixed at -80 mV, potential change stimulation to +20 mV was given and human Kv2.1 current generated in the inner membrane was measured. The current values of 21 clones were measured, the current value of a clone not expressing a peptide and only expressing human Kv2.1, was used as a control for analysis.

As a result, clone 4, 9, 16, and 46 exhibited antagonist activity, whereas, clone 30 exhibited agonist activity (Figure 4). Clone 32 exhibited neither one of the activities and was considered to exhibit a binder activity.

DNA sequences of clone 4, 9, 16, 30, 32, and 46 were determined and the amino acid sequences of peptides were determined.
Clone 4: DCLGRRRKCIPDNDKCCRPLLVCSRTHKGCWVMG (SEQ ID NO: 4)
Clone 9: DCLGSSRKCIPDNDKCCRPPLVCSRTHKSCTLFL (SEQ ID NO: 5)
Clone 16: DCLGRWRKCIPDNDKCCRPILVLCTPSG (SEQ ID NO: 6)
Clone 30: DCLGPLRKCIPDTTNAWQILYAVERTKKCWSAK (SEQ ID NO: 7)
Clone 32: DCLGSVRKCIPDNDKCCRPVLVCSRTHKFCFLVM (SEQ ID NO: 8)
Clone 46: DCLGYWRKCIPTTTNAWQVLYAVERHKECIMAK (SEQ ID NO: 9)

The sequences of clone 16, 30 and 46 were different from those of the original library. When new sequences were obtained, new research can be started based on these sequences. This is also an advantage in this method.

After the PERISS method was applied 5 times, the plasmid DNA sequence was analyzed by a next-generation sequencer. As a result, it was found that the above clone 4, 9, 30, 32, and 46 are contained (Figure 5).

In a conventional method, after the results by a next-generation sequencer were obtained, candidate peptides to be subjected to activity measurement are determined. Thereafter, the peptide is expressed in the periplasmic space by using e.g., pMal system (high-yield protein expression system with a highly soluble MBP (maltose binding protein) tag) manufactured by NEB, and purified by use of the MBP tag. This step includes, e.g., synthesis of oligo DNA of a peptide, recombination, sequencing, expression and purification. This process requires at least one month. If the time for preparation of samples for the next-generation sequencer is included, nearly 2 months is required to obtain the electrophysiological measurement results.

In this Example, it takes only two days after completion of the 5th PERISS process to obtaining electrophysiological measurement results. Even if the period of the following DNA sequencing according to the Sanger method is included, only one week is required. The method of the invention makes it possible to determine the activity of a candidate peptide at an extremely high speed, compared to the conventional PERISS method.

### Reference Example 1: Effect of GTx1-15 on THP-1 cell

THP-1 cells were cultured in RPMI-1640 containing 10% fetal bovine serum and supplemented with L-glutamine, penicillin, and streptomycin. For cytotoxicity assay, 3 × 10⁴ THP-1 cells were inoculated in a single well of a 96-well plate containing 80 µl of RPMI-1640 medium. Immediately after inoculation, 20 µl of a GTx1-15 solution was added to the well. The final concentration of GTx1-15 was either one of 1, 3, 10 and 30 µg/mL. As a control, RPMI-1640 medium was added to the wells in place of the GTx1-15 solution. Incubation was carried out for 24 hours, and thereafter, cytotoxicity was tested. A WST-1 solution (10 µl) was simply added to each well and incubated at 37°C for 2 hours. After completion of the incubation, absorption (absorbency) was measured at 450 nm/620 nm by a microplate reader.

For immunogenicity assay, 2.4 × 10⁶ THP-1 cells were inoculated in a single well of a 12-well plate containing 1.2 ml of RPMI-1640 medium. Immediately after inoculation, a GTx1-15 solution or a DNCB solution was added to individual wells. The final concentration of GTx1-15 was any one of 1, 3 and 10 µg/mL and the concentration of the DNCB (solution) serving as a positive control was 4 µg/mL. Incubation was carried out for 24 hours, and thereafter, THP1 cells were collected by centrifugation at 1000 g for 5 minutes. Then, the total RNA was extracted from the cells by use of RNeasy Mini Kit (QIAGEN). Using the total RNA as a template, cDNA was synthesized. The human CD80, CD86, CD54 and GAPDH expression levels were quantitatively obtained by quantitative PCR using specific primers to these.

GTx1-15 did not exhibit cytotoxicity against THP-1 cells at a concentration of 1 to 30 µg/ml (Figure 6). Significant expression of CD86, CD80, CD54 was not observed and confirmed that they have no immunogenicity (Figure 7).

### Reference Example 2: Temperature stability of GTx1-15

Thermal stability of GTx1-15 was checked by incubating a GTx1-15 sample (24.7 µM, 10 µg/100 µl) at various temperatures (20, 37, 50, 75 and 95°C) for 24 hours. Twenty four hours later, the sample (100 µl) was diluted with 400 µl of a 0.05% TFA solution, separated by use of a Superiorex ODS column (4.6 × 250 mM, Shiseido), and eluted with 0% to 30% (linear gradient) acetonitrile containing 0.05% TFA at a rate of 1 ml/min. The peak area representing the amount of GTx1-15 was calculated by use of the evaluation function of Unicorn software version 7.1.

GTx1-15 was maintained almost intact (without decomposition) from 20°C to 75°C, and 70% of GTx1-15 were maintained intact even at 95°C (Figure 8).

The inventors have confirmed that an ICK peptide such as GTx1-15 was not decomposed with a digestive enzyme; that the ICK peptide was not decomposed even if it was incubated in the rat plasma at 37°C for 24 hours, and that an ICK peptide intravenously administered can detected in blood for several hours (Kikuchi et al. International Journal of Peptides, 2015, as mentioned above). Further, GTx1-15 shows neither cytotoxicity nor immunogenicity and has stability at high temperature. In this sense, it can be said that GTx1-15 is a peptide having basic characteristics as a pharmaceutical. This suggests that a peptide obtained from a peptide library constructed with GTx1-15 as a template has the same characteristics. It is expected that not only GTx1-15 but also other ICK peptides commonly have the characteristics.

### Industrial Applicability

The present invention is useful in searching an agonist and antagonist of a membrane receptor, a drug transporter and an ion channel and searching a ligand substance for an orphan receptor, and in developing drugs.

All publications, patents and patent applications cited in the specification are incorporated herein in their entireties by reference.

### Sequence listing free text

SEQ ID NO: 1: synthetic peptide (library based on GTx1-15)
SEQ ID NO: 4: synthetic peptide (clone 4)
SEQ ID NO: 5: synthetic peptide (clone 9)
SEQ ID NO: 6: synthetic peptide (clone 16)
SEQ ID NO: 7: synthetic peptide (clone 30)
SEQ ID NO: 8: synthetic peptide (clone 32)
SEQ ID NO: 9: synthetic peptide (clone 46)
SEQ ID NO: 10 to 27: synthetic peptide (after concentration (5 rounds) plasmid DNA sequence)

## Claims

1. A method for screening for a polypeptide that acts on a target protein, comprising the steps of:
(1) providing a polynucleotide library constituted of a plurality of expression vectors that can be expressed in a gram-negative bacterium, wherein
the plurality of expression vectors each comprise a first polynucleotide encoding a polypeptide different from one another, a secretory signal sequence positioned upstream of the first polynucleotide, and a second polynucleotide encoding a target protein;
(2) transforming a gram-negative bacterium with the expression vector to express the polypeptide in a periplasmic space and the target protein on an inner membrane surface;
(3) contacting the polypeptide with the target protein in the periplasmic space;
(4) allowing the gram-negative bacterium to form a spheroplast to measure activity of the polypeptide on the target protein by an electrophysiological technique; and
(5) identifying a polypeptide that acts on the target protein based on the measured activity.

2. The method according to claim 1, wherein the gram-negative bacterium is *Escherichia coli.*

3. The method according to claim 1 or 2, wherein the target protein is a membrane protein.

4. The method according to claim 3, wherein the membrane protein is a membrane receptor, an ion channel or a transporter.

5. The method according to any one of claims 1 to 4, wherein the electrophysiological technique is a patch clamp technique.

6. The method according to any one of claims 1 to 5, comprising a step of concentrating the polynucleotide library for the first polynucleotide encoding a polypeptide that binds to a target protein in advance.

7. A method for producing a polypeptide that acts on a target protein, comprising
a step of identifying the polypeptide that acts on a target protein by the method according to any one of claims 1 to 6 and producing the identified polypeptide by gene recombination or chemical synthesis.

8. A method for preparing a library, comprising the steps of:
(1) applying an ODA analysis to a three-dimensional structure of a ligand for a known membrane protein to predict a site(s) that is likely to interact with another protein;
(2) preparing a plurality of polypeptides each having an amino acid sequence in which the predicted site(s) is randomized in the amino acid sequence of the ligand, or preparing a plurality of polynucleotides encoding the polypeptides; and
(3) preparing a library comprising the plurality of polypeptides or the plurality of polynucleotides.

9. The method according to claim 8, wherein the ligand is an ICK polypeptide.

10. The method according to claim 8 or 9, wherein the membrane protein is a membrane receptor, an ion channel or a transporter.

11. The method according to any one of claims 1 to 7, wherein the polynucleotide library is prepared by the method according to any one of claims 8 to 10.

12. A library comprising mutually different polypeptides having the following amino acid sequence, or a plurality of polynucleotides encoding the polypeptides,
DCLGXXRKCIPDNDKCCRPXLVCSRTHKXCXXXX (SEQ ID NO: 1).

13. The library according to claim 12, wherein each of the plurality of polynucleotides is inserted in an expression vector that can be expressed in a gram-negative bacterium.

14. The library according to claim 13, wherein the expression vector comprises the polynucleotide, a secretory signal sequence arranged upstream of the polynucleotide and a polynucleotide encoding a target protein.

15. A polypeptide having the amino acid sequence set forth in any one of SEQ ID NOs: 4 to 9.
